(19) Europäisches Patentamt
European Patent Office
Office européen des brevets

(11) Publication number: 0 361 842 A2

(12) **EUROPEAN PATENT APPLICATION**

(21) Application number: 89309752.7

(22) Date of filing: 26.09.89

(51) Int. Cl.5: A61L 15/00

(30) Priority: 26.09.88 US 249540

(43) Date of publication of application: 04.04.90 Bulletin 90/14

(84) Designated Contracting States: BE DE ES FR GB IT NL

(71) Applicant: ARCO CHEMICAL TECHNOLOGY INC.
3 Christina Centre Suite 902 201 N Walnut Street
Wilmington Delaware 19801(US)

(72) Inventor: Le-Khac, Bi
1210 Birmingham Road
West Chester, PA 19382(US)
Inventor: Pilcicki, Edward J.
1224 Third Avenue
Gilbertsville, PA 19525(US)

(74) Representative: Cropp, John Anthony David et al
MATHYS & SQUIRE 10 Fleet Street
London, EC4Y 1AY(GB)

(54) **Multi-layered absorbent articles and process for their production.**

(57) A layered article with high fluid retention properties comprising A) at least one layer of a non-woven web having from 5% to 100% hydrophilic fibers and from 95% to 0% other fibers; and B) at least one layer of backing material integrally attached to the non-woven web; wherein the hydrophilic fibers are formed from (a) a copolymer having 25 mole % to 75 mole % of an alpha, beta-unsaturated monomer bearing at least one pendant carboxylic acid or carboxylate salt group, and 75 mole % to 25 mole % of a copolymerizable comonomer, wherein from about 20% to 80% of the pendant groups are carboxylic acid groups and from about 80% to 20% of the pendant groups are carboxylate groups and (b) a cross-linking monomer selected from heterocyclic carbonate compounds and compounds having at least two free hydroxyl groups.

EP 0 361 842 A2

# MULTI-LAYERED ABSORBENT ARTICLES AND PROCESS FOR THEIR PRODUCTION

## Background of the Invention

This application is related to US 4,731,067 and European patent application 88306867.8 published as EP-A-0301804, the disclosures of which are incorporated-by-reference herein.

## Field of the Invention

This invention relates to multi-layered superabsorbent articles exhibiting both high strength and excellent liquid absorption properties. The multi-layered articles, or laminates, of this invention are prepared by bonding a non-woven mat of highly absorbent fibers to a suitable substrate. The laminates are useful per se or may be combined with other materials to form a variety of structures, such as diapers, sanitary napkins, incontinency products, and others for the absorption of fluids.

## Information Disclosure Statement

U.S. 4,260,443 discloses laminates of tissue-like materials having liquid absorbing agents fixed in position between the lamina. The liquid absorbing agent is a powder flake or fiber, which is distributed between the layers and fixed in position by applying water at spaced points to moisten the water absorbing powder and cause it to serve as an adhesive at those points.

U.S. 4,293,609 discloses flexible absorbent laminates made from crushed films of water-swellable hydrophilic polymer with layers of wicking substrates. The laminate of this invention is said to have a central substantially discontinuous and crushed film consisting of a water swellable hydrophilic polymer as its absorbent medium.

U.S. 4,297,410 discloses an absorbent material comprising a non-woven fabric on which a highly absorbent hydrophilic polymer substrate substance is scattered and which is thermally set.

U.S. 4,330,021 discloses an absorbent article comprising a cover sheet and a backing sheet in which the cover and backing sheets are attached discontinuously to form pockets in which fluid absorbent material is stored.

U.S. 4,467,012 discloses a laminated product in which a small amount of a polyhydric alcohol is added to a hydrolyzed starch polyacrylonitrile graft copolymer and disposed between a pair of backing tissues in the presence of heat and pressure.

U.S. 4,454,055 discloses a dry, solid, water-swellable absorbent composition prepared by blending together a water-insoluble absorbent polymer and an extender material.

Generally, the prior art products suffer from the disadvantages of having discontinuous pockets of absorbent materials as well as problems associated with migration of powder or gel-like particles in the laminate substrate. In addition, the prior art products are deficient in flexibility and other fabric-like properties.

## Summary of the Invention

According to the present invention, it has been found that superabsorbent laminated articles may be prepared by bonding a fibrous mat prepared from superabsorbent non-woven fibers to suitable backing materials. The backing materials may themselves be hydrophilic or hydrophobic as dictated by the end-use of the invention.

It is an object of this invention to provide superabsorbent laminates with excellent absorption speed and capacity.

It is a further object of this invention to provide superabsorbent laminates having excellent conformability, drapability and other fabric-like properties.

It is a further object of this invention to provide a high productivity process for the production of superabsorbent laminated articles.

This invention provides a layered article with high fluid retention properties comprising at least one layer of non-woven web having from 5% to 100% hydrophilic fibers and from 95% to 0% other fibers in

conjunction with at least one other layer of backing material integrally attached to the non-woven web.

The hydrophilic fibers are formed from a copolymer and at least one crosslinking agent selected from heterocyclic carbonate compounds and compounds having at least two hydroxyl groups. The copolymer comprises 25 mole % to 75 mole % of an alpha, beta unsaturated monomer bearing at least one pendant carboxylic acid or carboxylate salt group, and 75 mole % to 25 % mole of at least one copolymerizable comonomer. From about 20% to about 80% of the pendant groups which are introduced from the α, β-unsaturated monomer are carboxylic acid groups and from about 80% to about 20% of the pendant groups are carboxylate salt groups.

The copolymers are formed into filaments by conventional fiber forming means and deposited onto a moving web or screen for take-up. The non-woven web may then be separately embossed to the substrate materials. Alternatively, the non-woven web fibers may be deposited directly upon the substrate material and bonded thereto by application of heat, pressure or both.

## Detailed Description of the Invention

The copolymer of the polymer composition may contain about 25-75 mole percent recurring units of at least one alpha, beta-unsaturated monomer bearing at least one pendant unit selected from carboxylic acid units and derivatives of carboxylic acid units, and about 75-25 mole percent recurring units of at least one monomer copolymerizable therewith, wherein about 20-80 mole percent of the total pendant units introduced through the recurring alpha, beta-unsaturated monomer units are carboxylic acid units or which are converted into carboxylic acid units, and wherein about 80-20% of the total pendant units are carboxylate salt units or which are converted into carboxylate salt units. Preferably, the copolymer will contain about 35-65 total mole percent of recurring units of at least one alpha, beta-unsaturated monomer and about 65-35 total mole percent of at least one copolymerizable monomer. More preferably, the comonomers of the copolymer will be present in equimolar proportions.

Suitable hydroxyl-containing crosslinking units include one or more compounds having at least two hydroxyl groups, such as alkylene glycols of 2-10 carbon atoms and ethers thereof, cyclic alkylene glycols, bisphenol A, hydroxy alkylene derivatives of bisphenol A, hydroquinone, phloroglucinol, hydroxy alkylene derivatives of diphenols, glycerol, erythritol, pentaerythritol, monosaccharides and other compounds specified hereinafter.

Typical alpha, beta-unsaturated monomers useful in the invention include maleic acid, crotonic acid, fumaric acid, mesaconic acid, the sodium salt of maleic acid, the sodium salt of 2-methyl-2-butene dicarboxylic acid, the sodium salt of itaconic acid, maleamic acid, maleamide; N-phenyl maleimide, maleimide, maleic anhydride, fumaric anhydride, itaconic anhydride, citraconic anhydride, methyl itaconic anhydride, ethyl maleic anhydride, diethylmaleate, methylmaleate, and the like, and any mixtures thereof.

Any suitable copolymerizable comonomer can be employed. Suitable copolymerizable comonomers include ethylene, propylene, isobutylene, $C_1$ to $C_4$ alkyl (meth)acrylates, vinyl acetate, methyl vinyl ether, isobutyl vinyl ether, and styrenic compounds having the formula:

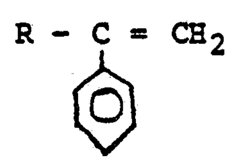

wherein R represents hydrogen or an alkyl group having from 1 to 6 carbon atoms and wherein the benzene ring may be substituted with low molecular weight alkyl or hydroxy groups.

Typical $C_1$ to $C_4$ alkyl acrylates include methyl acrylate, ethyl acrylate, isopropyl acrylate, n-propyl acrylate, n-butyl acrylate, and the like, and any mixtures thereof. Suitable $C_1$ to $C_4$ alkyl methacrylates include methyl methacrylate, ethyl methacrylate, isopropyl methacrylate, n-propylmethacrylate, n-butyl methacrylate, and the like, and any mixtures thereof. Suitable styrenic compounds include styrene, alpha-methylstyrene, p-methylstyrene, t-butyl styrene, and the like, and any mixtures thereof.

The pendant units on the alpha, beta-unsaturated monomer, will determine what, if any, additional reactions must be carried out to obtain a copolymer having the requisite pendant units necessary to produce water-absorbing polymer compositions useful in the invention having about 20-80 percent pendant groups such as carboxylic acid units and about 80 to about 20 percent pendant carboxylate salt units.

Preferably, both units are present in an amount of from about 30 to about 70 percent.

In general, if the alpha, beta-unsaturated monomer bears only carboxylic acid amide, carboxylic acid imide, carboxylic acid anhydride, carboxylic acid ester groups, or mixtures thereof, it will be necessary to convert at least a portion of such carboxylic acid derivative groups to carboxylic acid groups by, for example, a hydrolysis reaction. If the alpha, beta-unsaturated monomer bears only carboxylic acid salt groups, acidification to form carboxylic acid groups will be necessary.

Similarly, the final copolymer must contain about 80-20 percent pendant carboxylate salt units. Accordingly, it may be necessary to carry out a neutralization reaction. Neutralization of carboxylic acid groups with a strong organic or inorganic base such as NaOH, KOH, ammonia, ammonia-in-water solution, or organic amines will result in the formation of carboxylate salt units, preferably carboxylate metal salt units.

The sequence and the number of reactions (hydrolysis, acidification, neutralization, etc.) carried out to obtain the desired functionality attached to the copolymer backbone are not critical. Any number and sequence resulting in a final copolymer which possesses about 20-80 percent pendant carboxylic acid units and about 80-20 percent pendant carboxylate salt units is suitable.

One copolymer particularly suitable for use in forming superabsorbent webs in accordance with the invention is a copolymer of maleic anhydride and isobutylene. Another is maleic anhydride and styrene. Suitable copolymers will have peak molecular weights of from about 5,000 to about 500,000 or more. The copolymers of maleic anhydride and isobutylene and/or styrene can be prepared using any suitable conventional methods. Maleic anhydride/isobutylene copolymers are also commercially available from Kuraray Isoprene Chemical Company, Ltd., Tokyo, Japan, under the trademark ISOBAM. ISOBAM copolymers are available in several grades which are differentiated by viscosity molecular weight: ISOBAM-10, 160,000 to 170,000; ISOBAM-06, 80,000 to 90,000; ISOBAM-04, 55,000 to 65,000; and ISOBAM-600, 6,000 to 10,000; and ISOBAM 18, 180,000 to 300,000.

To produce water-absorbing polymer compositions useful in the invention, at least one copolymer as described above, and at least one crosslinking compound selected from heterocyclic carbonate compounds and compounds having at least two hydroxyl groups are blended such that the water-absorbing composition contains in weight percent about 80-99.5 total copolymer and about 0.5-20 total crosslinking compound. Preferably, the composition will contain about 90-99 weight percent total copolymer and about 1-10 weight percent total crosslinking agent.

Any suitable organic compound selected from heterocyclic carbonate compounds and compounds having at least two hydroxyl groups and having a relatively low molecular weight, less than 1,000, can be employed as a crosslinking agent for the copolymers.

Suitable crosslinking compounds include ethylene carbonate, propylene carbonate, 1,2-butylene carbonate, 2,3-butylene carbonate, phenyl ethylene carbonate, ethylene glycol, propylene glycol, trimethylene glycol, 1,4-butanediol, 1-methyl-1, 3-propane diol, neopentyl glycol, 1,5-pentane diol, diethylene glycol, dipropylene glycol, 1,4-cyclohexane dimethanol, 1,1,1-tris(hydroxymethyl1)ethane, Bisphenol A, 1,4-bis-(beta-hydroxyethoxy) bisphenol, hydroquinone, phloroglucinol, glycerol, erythritol, pentaerythritol, meso-erythritol, 1,7-dihydroxysedoheptulose, sucrose, natural monosaccharides, and the like, including any mixtures thereof.

The water-absorbing compositions of this invention are particularly well suited for being made into fibers because of the wide time and temperature ranges over which they can be shaped. More specifically, the water-absorbing compositions of this invention possess shelf lives on the order of 6 months to two years and can be formulated to cure at temperatures within the range of from about 140°C to about 250°C or higher. Hence, the water-absorbing compositions of this invention can be easily made into fibers using conventional fiber-forming methods and equipment.

The fibers of this invention may be prepared by the dry spinning process disclosed in U.S. 4,731,067 or by the air attenuation process of EP-A-0301804. Alternative fiber-forming methods may be employed as deemed desirable by one skilled in the art.

The water-absorbing compositions of this invention and articles of manufacture into which the compositions are incorporated are suitable for use in a wide range of absorptive functions. In general, the articles into which the water-absorbing compositions are incorporated serve the function of supporting the composition and presenting it in a form adapted for absorptive end use. Means to support and present the composition for absorptive use include, but are not meant to be limited to bandages, surgical and dental sponges, tampons, sanitary napkins and pads, disposable diapers, meat trays, pads for absorption of perspiration, and the like.

By way of summary, in one aspect of the present invention, there is provided a layered article with high fluid retention properties comprising A) at least one layer of a non-woven web having from 5% to 100%

hydrophilic fibers and from 95% to 0% other fibers; and B) at least one layer of backing material integrally attached to the non-woven web; wherein the hydrophilic fibers are formed from (a) a copolymer having 25 mole % to 75 mole % of an alpha, beta-unsaturated monomer bearing at least one pendant carboxylic acid or carboxylate salt group, and 75 mole % to 25 mole % of a copolymerizable comonomer, wherein from about 20% to 80% of the pendant groups are carboxylic acid groups and from about 80% to 20% of the pendant groups are carboxylate salt groups, and (b) a cross-linking monomer selected from heterocyclic carbonate compounds and compounds having at least two free hydroxyl groups.

Preferred features of said article are as follows:

the backing material is a woven, non-woven, or knit fabric;

the backing material is a cellulosic, olefinic, polyester, acrylic, or polyamide non-woven sheet;

the non-woven web is integrally attached to the backing material by one or more of the following methods: thermal fusion, adhesive bonding and mechanical entangling;

the non-woven web may be formed from continuous filaments of the hydrophilic fibers or it may be formed from staple fibers; or both;

the layered article is embossed;

at least one layer of backing material is impervious to moisture;

at least one backing material is elastic;

the layered article has a first layer of backing material on one face of the non-woven web and a second layer of the same or different backing material on the opposite face of the non-woven web and the layers are preferably thermally bonded;

the non-woven web comprises fibers of an isobutylene/maleic anhydride copolymer.

The invention also provides an absorbent layered article comprising at least one layer of a non-woven fibrous mat having a free swell of at least 40 g/g and at least one second layer integrally attached thereto.

Preferred features of this article are:

the non-woven fibrous mat is bonded to a woven or non-woven substrate;

the non-woven fibrous mat is composed of continuous filaments;

the non-woven web is thermally bonded to the substrate.

The invention further provides a layered article comprising at least one layer of a hydrophilic non-woven web of fibers formed by cross-linking an isobutylene/maleic anhydride copolymer modified to bear pendant carboxylic acid and carboxylate salt groups and at least one layer of a non-woven substrate.

Preferred features of this article are:

the copolymer is cross-linked with propylene carbonate, butanediol, pentaerythritol, or a mixture thereof;

the hydrophilic non-woven web is sandwiched between two layers of cellulosic paper;

the non-woven substrate is polypropylene or polyethylene;

the hydrophilic non-woven web comrises randomly laid continuous filaments.

The invention also provides a process for preparing a layered article which comprises:

(a) preparing an 30-60% solids aqueous solution of a polymeric composition which is super absorbent upon curing;

(b) extruding the solution into fibers;

(c) partially drying and attenuating the fibers;

(d) collecting the fibers on a foraminous surface in the form of a web;

(e) overlaying the web with at least one layer of a backing material;

(f) bonding the web to the backing material; and

(g) curing the fibers at any time after extruding to form the superabsorbent composition.

Preferred features of this process are:

the process further comprises the step of curing the fibers prior to collecting on the foraminous surface;

the process further comprises the step of curing the web prior to the bonding step;

the step of curing and compacting the web is effected prior to overlaying;

the solution is extruded into a column having heated gas flowing therethrough for drying the fibers and removing the vapors;

the fibers are attenuated by air jets;

a pattern is embossed onto the web;

the solution is extruded as continuous filaments and the continuous filaments are collected on the foraminous surface;

the fibers are collected directly on the backing material;

the bonding is effected by hot pressing.

The invention is now illustrated by the following Examples:

Demand Absorption Test

The test procedure is as follows: A piece of hydrophilic paper liner (cut out of Lipton tea bag, approximately 2 square inches) was placed over the opening of the sample platform of a Demand Absorption Tester from Scientific Machine and Supply Co.. The level of the burette was then adjusted to bring the brine level up touching the liner and making the liner barely damp. A sample of absorbent (mat, laminate, or powder) approximately one square inch in area and 0.15-0.20g in weight was placed over the opening on the sample platform on the liner. Fluid wicking started as soon as the burette and air bleed stopcocks were opened. The timer was activated when the first bubbles bled into the burette. The time was recorded at each half milliliter of brine absorbed. The volume of brine absorbed was divided by the initial sample weight to obtain absorption in grams of brine/gram of sample. Plots of demand absorption speeds versus times are used to find the absorption speed (grams of brine/one gram of sample/second) from the initial slope of the curve.

Swell Index

This test procedure is described in U. S. 4,454,055, the teachings of which are incorporated herein by reference thereto. The test procedure and equipment used herein were modified slightly as compared to the procedure and equipment described in U. S. 4,454,055.

To determine the Free Swell Index at atmospheric (room) pressure, about 0.2 to 0.3 g of the water-absorbing composition to be tested is placed in an empty W-shaped tea bag. The tea bag containing the composition is immersed in brine (0.9 wt. % NaCl) for 10 minutes, removed and allowed to sit on a paper towel for 30 seconds to remove surface brine. The Swell Index of the composition, that is, the units of liquid absorbed per each unit of sample is calculated using the following formula:

$$\text{Free Swell Index} = \frac{\text{Weight of Wet Composition}}{\text{Weight of Dry Composition}} - 1$$

To determine Swell Index under pressure, the following modified procedure was used.

After the tea bag containing the sample composition is immersed in brine and surface brine is removed, it is immediately placed in a 16 cm ID Buchner funnel fitted with a 2000 ml sidearm vacuum filter flask and connected to a manometer. Then, a piece of dental dam rubber sheeting is securely fixed over the mouth of the funnel such that the sheeting just rests on the tea bag. Next, a vacuum sufficient to create the desired pressure is drawn on the flask for a period of five minutes, and, the Swell Index under pressure is calculated using the above formula.

EXAMPLE 1:

A water-absorbing composition useful in making laminated products of this invention was prepared by mixing 65 lbs of an isobutylene/maleic anhydride copolymer (ISOBAM 10), (Kuraray Isoprene Chemical Co., Ltd.) and 107.3 lbs of demineralized water in a Ross mixer and heating to about 90°C. 33.6 lbs of a 50% aqueous solution of NaOH was added slowly to the mixer over a 45 minute period. 4.55 lbs of propylene carbonate were introduced into the mixer and the agitation was continued for about 10 hours at 90°C to complete the reaction. A fibrous mat was then prepared from this aqueous solution via the air attenuation process of U.S.S.N. 079,312. The resulting fibers had diameters of about 10-25 microns. The fibrous mat produced from this composition was cured for 20 minutes at 210°C. This sample had a free swell index of 45 g/g, a 0.5 psi swell index of 30 g/g, and an absorption speed of 0.282 g/g/sec.

EXAMPLE 2:

The cured fibrous mat of Example 1 was bonded to nonwoven cellulosic paper on an embossing pattern screen. The whole system (fibrous mat, paper and screen) was hot pressed at 200°F for 2 minutes under about 2000 to 5000 psi. Examples 2A and 2B were prepared with a single layer of paper. The density of the

fibrous mat was lower for Example 2B thereby yielding a lower percentage of fiber in the finished article. The absorption speeds of the fibrous mat of Example 1 and the resultant laminates (2A and 2B) of this Example 2 were measured by using a demand absorption tester with 0.9% brine solution. The free swell and 0.5 psi swell indices of these samples were also measured. The results of these tests are shown in Table 1.

TABLE 1

| EXAMPLE | 1 | 2A | 2B |
|---|---|---|---|
| FIBROUS MAT (WT. %) | 100 | 90 | 78 |
| FREE SWELL INDEX (G/G) | 45.0 | 45.6 | 43.3 |
| 0.5 PSI SWELL INDEX (G/G) | 30.0 | 29.3 | 28.7 |
| ABSORPTION SPEED (G/G/SEC) | 0.282 | 0.673 | 1.118 |

EXAMPLE 3

1143.8 g of ISOBAM 10 and 2050 g of demineralized water were charged to a one gallon stirred reactor and heated to about 90°C. 652.8 g of 50% NaOH aqueous solution was added slowly to the reactor over a one hour period with agitation. After the addition of the sodium hydroxide solution, agitation was continued for about 10 hours at 90°C to complete the reaction. 22.8 g of butanediol and 91.5 g of pentaerythritol were then added to the reactor with agitation for one hour at 90°C. Fibrous mats were then prepared from this aqueous solution using the attenuation process of U.S. S.N. 079,312. The resultant fibrous mats were divided into two portions and separately cured by heating in a hot air circulation oven at 180°C for 10 minutes (3A) and 15 minutes (3B). Testing results are shown in Table 2.

TABLE 2

| EXAMPLE | 3A | 3B |
|---|---|---|
| CURE TEMP. (°C) | 180 | 180 |
| CURE TIME (MIN) | 10 | 15 |
| FREE SWELL INDEX (G/G) | 84.6 | 45.5 |
| 0.5 PSI SWELL INDEX (G/G) | 57.8 | 30.8 |
| ABSORPTION SPEED (G/G/SEC) | 0.04 | 0.28 |

EXAMPLE 4

The fibrous mat of Example 3B, cured at 180°C for 15 minutes, was used to prepare laminated product with a 19 g/sg yd polypropylene nonwoven fabric (Scott, Brand #6723) using the procedure of Example 2. A single nonwoven layer was used to prepare Example 4A and Example 4B; the mat of Example 4B as less dense resulting in a lower percent fiber in the finished article. The effects of the hydrophobic nonwoven fabric on the absorbent properties and absorption speed of Example 3B are shown in Table 3.

TABLE 3

| EXAMPLE | 3B | 4A | 4B |
|---|---|---|---|
| FIBROUS MAT (Wt.%) | 100 | 80 | 66 |
| FREE SWELL (G/G) | 45.5 | 33.4 | 30.5 |
| 0.5 PSI (G/G) | 30.8 | 21.0 | 19.2 |
| SPEED (G/G/SEC) | 0.28 | 1.30 | 0.889 |

EXAMPLE 5:

The experiment of Example 4 was repeated using the fibrous mat of Example 3A cured at 180° C for 10 minutes and a single sheet of polypropylene nonwoven fabric. The results are shown in Table 4.

TABLE 4

| EXAMPLE | 3A | 5 |
|---|---|---|
| FIBROUS MAT (Wt. %) | 100 | 85 |
| FREE SWELL INDEX (G/G) | 84.6 | 63.1 |
| 0.5 PSI SWELL INDEX (G/G) | 57.8 | 43.2 |
| ABSORPTION SPEED (G/G/SEC) | 0.04 | 0.393 |

EXAMPLE 6:

1143.8 g OF ISOBAM 10 and 1806.5 g of demineralized water were charged to a one gallon stirred reactor and heated to about 90° C. 593.5 g of 50% NaOH aqueous solution were added slowly to the reactor over a one hour period with agitation. After the addition of the sodium hydroxide solution, agitation was continued for about 10 hours at 90° C to complete the reaction. 137.3 g of pentaerythritol were then added to the reactor with agitation for one hour at 90° C. A fibrous mat was then prepared from this aqueous solution using the air attentuation process. One portion of this uncured fibrous mat was used to make laminated product with one layer of nonwoven cellulosic paper (16 Gms/Sq. Yd.). Embossing was carried out at 374° F for 5 minutes to cure and emboss the laminate at the same time in the press.

The resultant laminated product (80% fibrous mat and 20% paper) had a free swell index of 46.6 g/g and 0.5 psi swell index of 29.3 g/g and an absorption speed of 0.83 g/g/sec.

EXAMPLE 7

A portion of fibrous mat produced in Example 6 was cured at 160° C for 30 minutes then laid between two layers of nonwoven cellulosic paper (16 Gms/Sq. Yd.). This system was embossed with an embossing pattern screen on each side at 200° F for 2 minutes in the press.

The resultant laminated product containing about 72% fibrous mat and 28% paper had an absorption speed of 1.232 g/g/sec. The free swell and 0.5 psi swell indices were 45.7 g/g and 28.5 g/g, respectively.

Example 8

The solution of Example 6 is extruded into fibers through a spinneret at a rate between 0.05-.60 grams/hole/min into a heated dry spinning column maintained at 60° C to 300° C. These fibers are drawn into an air aspirator (gun) set at 10 to 150 psi air pressure. Continuous dry fibers are blown from the gun

onto the belt of a curing oven moving at 1.5-60 feet/min. The temperature of the oven is set at 150-260 °C. with curing times from 1-40 min. After emerging from the curing oven, the fibers are passed through the embosser with the selected liner. The embosser is set between 25 °C and 260 °C and 1,000-30,000 lbs. pressure. The resultant product is either cut into sheets or rolled on a roller.

## Claims

1. A layered article with high fluid retention properties comprising A) at least one layer of a non-woven web having from 5% to 100% hydrophilic fibers and from 95% to 0% other fibers; and B) at least one layer of backing material integrally attached to the non-woven web; wherein the hydrophilic fibers are formed from (a) a copolymer having 25 mole % to 75 mole % of an alpha, beta-unsaturated monomer bearing at least one pendant carboxylic acid or carboxylate salt group, and 75 mole % to 25 mole % of a copolymerizable comonomer, wherein from about 20% to 80% of the pendant groups are carboxylic acid groups and from about 80% to 20% of the pendant groups are carboxylate salt groups, and (b) a cross-linking monomer selected from heterocyclic carbonate compounds and compounds having at least two free hydroxyl groups.

2. The layered article of claim 1 in which the backing material is a woven, nonwoven, or knit fabric.

3. The layered article of claim 1 or claim 2 in which the backing material is a cellulosic, olefinic, polyester, acrylic, or polyamide nonwoven sheet.

4. A layered article as claimed in any one of claims 1 to 3 in which the non-woven web is thermally fused, adhesively bonded and/or mechanically entangled with the backing material.

5. A layered article as claimed in any one of claims 1 to 4 in which the non-woven web is formed from continuous filaments of the hydrophilic fibers and/or is formed from staple fibers.

6. A layered article as claimed in any one of claims 1 to 5 which is embossed.

7. A layered article as claimed in any one of claims 1 to 6 in which at least one layer of backing material is impervious to moisture.

8. A layered article as claimed in any one of claims 1 to 7 in which at least one backing material is elastic.

9. A layered article as claimed in any one of claims 1 to 8 having a first layer of backing material on one face of the non-woven web and a second layer of the same or different backing material on the opposite face of the non-woven web.

10. A layered article as claimed in claim 9 in which the layers are thermally bonded.

11. A layered article as claimed in any one of claims 1 to 10 in which the non-woven web comprises fibers of an isobutylene/maleic anhydride copolymer.

12. An absorbent layered article comprising at least one layer of a nonwoven fibrous mat having a free swell of at least 40 g/g. and at least one second layer integrally attached thereto.

13. An article as claimed in claim 12 in which the non-woven fibrous mat is bonded to a woven or non-woven substrate.

14. An article as claimed in claim 12 or claim 13 in which the non-woven fibrous mat is composed of continuous filaments.

15. An article as claimed in any one of claims 12 to 14 in which the non-woven web is thermally bonded to the substrate.

16. A layered article comprising at least one layer of a hydrophilic non-woven web of fibers formed by cross-linking an isobutylene/maleic anhydride copolymer modified to bear pendant carboxylic acid and carboxylate salt groups and at least one layer of a nonwoven substrate.

17. An article as claimed in claim 16 in which the copolymer is cross-linked with propylene carbonate, butanediol, pentaerythritol, or a mixture thereof.

18. An article as claimed in claim 16 or claim 17 in which the hydrophilic non-woven web is sandwiched between two layers of cellulosic paper.

19. An article as claimed in claim 16, 17 or 18 in which the non-woven substrate is polypropylene or polyethylene.

20. An article as claimed in any one of claims 16 to 19 in which the hydrophilic, non-woven web comprises randomly laid continuous filaments.

21. A process for preparing a layered article which comprises:

(a) preparing an 30-60% solids aqueous solution of a polymeric composition which is superabsorbent upon curing;

(b) extruding the solution into fibers;

(c) partially drying and attenuating the fibers;

(d) collecting the fibers on a foraminous surface in the form of a web;

(e) overlaying the web with at least one layer of a backing material;

(f) bonding the web to the backing material; and

(g) curing the fibers at any time after extruding to form the superabsorbent composition.

22. The process of claim 21 further comprising the step of curing the fibers prior to collecting on the foraminous surface.

23. The process of claim 21 further comprising the step of curing the web prior to the bonding step.

24. A process as claimed in claim 21, 22 or 23 further comprising the step of curing and compacting the web prior to overlaying.

25. A process as claimed in any one of claims 21 to 24 in which the solution is extruded into a column having heated gas flowing therethrough for drying the fibers and removing the vapors.

26. A process as claimed in any one of claims 21 to 25 in which the fibers are attenuated by air jets.

27. A process as claimed in any one of claims 21 to 26 further comprising embossing a pattern onto the web.

28. A process as claimed in any one of claims 21 to 27 comprising extruding the solution as continuous filaments and collecting the continuous filaments on the foraminous surface.

29. A process as claimed in any one of claims 21 to 28 further comprising collecting the fibers directly on the backing material.

30. A process as claimed in any one of claims 21 to 29 wherein the bonding is effected by hot pressing.